# EUROPEAN PATENT SPECIFICATION

(11) **EP 1 438 045 B1**
(45) Date of publication and mention of the grant of the patent: **14.02.2007**
(21) Application number: 02786300.0
(22) Date of filing: 22.10.2002
(51) Int. Cl.: A61K 31/4045, A61P 3/04

(54) **USE OF INDOLE AND INDOLINE DERIVATIVES IN THE TREATMENT OF OBESITY OR FOR THE REDUCTION OF FOOD INTAKE**
VERWENDUNG VON INDOL- UND INDOLIN-DERIVATEN BEI DER BEHANDLUNG VON ADIPOSITAS ODER ZUR REDUZIERUNG DER NAHRUNGSAUFNAHME
UTILISATION DE DERIVES D'INDOLE ET D'INDOLINE POUR LE TRAITEMENT DE L'OBESITE OU LA REDUCTION DE L'APPORT ALIMENTAIRE

(30) Priority: 23.10.2001 SE 0103539; 14.12.2001 US 340599 P
(43) Date of publication of application: 21.07.2004
(73) Proprietor: BIOVITRUM AB, 112 76 Stockholm (SE)
(72) Inventor: CALDIROLA, Patrizia, S-756 46 Uppsala (SE)
(86) International application number: PCT/SE2002/001929
(87) International publication number: WO 2003/035061

(56) References cited:
- EP-A1- 0 375 133
- WO-A1-92/07829
- GB-A- 2 341 549
- US-A- 4 576 959
- BENTLEY J.C. ET AL.: 'Effect of the 5-HT6 antagonist, Ro 04-6790 on food consumption in rats trained to a fixed feeding regime' BRITISH JOURNAL OF PHARMACOLOGY vol. 126, no. SUPPL., 1999, page 66P, XP002960509
- DOURISH COLIN T.: 'Multiple serotonin receptors: Opportunities for new treatments for obesity' OBESITY RESEARCH vol. 3, no. SUPPL. 4, November 1995, pages 449S - 462S, XP002948186

## Description

### Technical Field

The present invention relates to the use of indole and indoline derivatives, which bind selectively to 5-HT₆ receptors, in the manufacture of a medicament for the treatment of obesity.

### Background Art

Obesity is a condition characterized in an increase in body fat content resulting in excess body weight above accepted norms. Obesity is the most important nutritional disorder in the western world and represents a major health problem in all industrialized countries. This disorder leads to increased mortality due to increased incidences of diseases such as cardiovascular disease, digestive disease, respiratory disease, cancer and NIDDM (type II diabetes). Searching for compounds which reduce body weight has been going on for many decades. One line of research has been activation of serotonergic systems, either by direct activation of serotonin receptor subtypes or by inhibiting serotonin re-uptake. The exact receptor subtype profile required is however not known.

Serotonin (5-hydroxytryptamine or 5-HT), a key transmitter of the peripheral and central nervous system, modulate a wide range of physiological and pathological functions, including anxiety, sleep regulation, aggression, feeding and depression. Multiple serotonin receptor subtypes have been identified and cloned. One of these, the 5-HT₆ receptor, was cloned by several groups in 1993 (Ruat et al. (1993) Biochem. Biophys. Res. Commun., 193: 268-276; Sebben et al. (1994) NeuroReport 5: 2553-2557) This receptor is positively coupled to adenylyl cyclase and displays affinity for antidepressants such as clozapine. Recently, the effect of 5-HT₆ antagonist and 5-HT₆ antisense oligonucleotides to reduce food intake in rats has been reported (Bentley et al. (1999) Br. J. Pharmac. Suppl 126: P66; Bentley et al. (1997) J. Psychopharmacol. Suppl. A64: 255; Woolley, M.L. et al. (2001) Neuropharmacology 41: 210-219).

U.S. patent No. 6,187,805 (see also Russell, M.G.N. et al. (2001) "N-Arylsulfonylindole Derivatives as Serotonin 5-HT₆ Receptor Ligands", J. Med. Chem. *in press*) discloses indole and indoline derivatives as ligands selective for the 5-HT₆ receptors, and of proposed value in the treatment or prevention of CNS disorders, including Alzheimer's disease, Parkinson's disease, schizophrenia, depression and anxiety. However, it has not been disclosed that such derivatives are useful for the treatment of obesity.

### Brief description of the Drawings

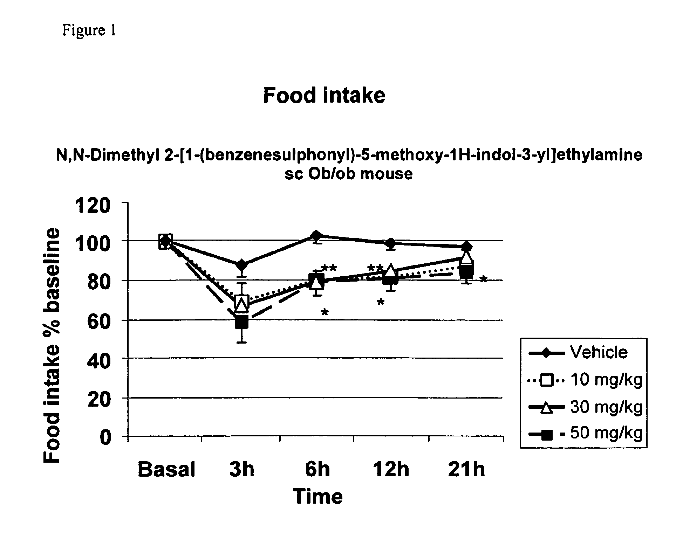

Figure 1 is a graph depicting the effect on food intake in obese mice by administration of a compound according to the invention.

### Disclosure of the Invention

It has been found that 5-HT₆ receptor antagonists, belonging to the class of indole or indoline derivatives disclosed in US 6,187,805, reduce food intake and body weight. Consequently, the present invention provides the use of compounds in the manufacture of a medicament for the treatment or prophylaxis of obesity in mammals, including humans. The invention comprises the use of a compound of formula II 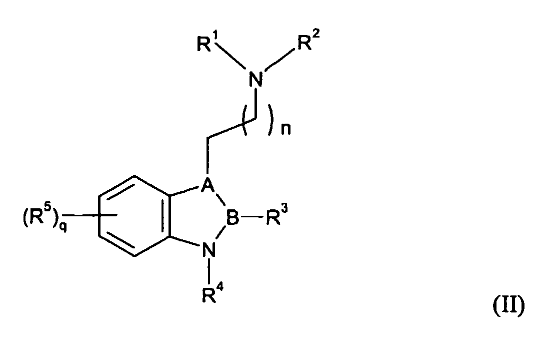 wherein
n is 1 or 2;
q is 0,1,2,3 or 4;
R¹ and R² are identical and independently represent hydrogen or methyl, or together complete a pyrrolidinyl, piperidinyl, piperazinyl or 4- methylpiperazinyl ring comprising the nitrogen atom to which R¹ and R² are attached;
R³ represents hydrogen, C₁₋₆ alkyl, C₂₋₆ alkenyl, C₂₋₆ alkynyl, aryl(C₁₋₆)alkyl, aryl, heteroaryl, arylcarbonyl, heteroarylcarbonyl, C₁₋₆ alkylcarbonyl, or C₁₋₆ alkoxycarbonyl;
R⁴ represents arylsulphonyl, heteroarylsulphonyl, C₁₋₆ alkylsulphonyl, di(C₁₋₆)alkylaminosulphonyl, arylcarbonyl, C₁₋₆ alkylcarbonyl, heteroarylcarbonyl or C₁₋₆ alkoxycarbonyl;
each R⁵ independently represents hydrogen, hydroxy, C₁₋₆ alkoxy, aryl(C₁₋₆)alkoxy, nitrile or halogen;
   and
A-B represents C=C or CH-CH, in the manufacture of a medicament for treatment of obesity

In formula II one or more substituents may be present on any alkyl or aryl group represented by any of R¹ -R⁵, or on any alkyl or aryl moiety of a group represented by any of R¹ -R⁵. Preferred substituents include C₁₋₆ alkyl, halogen, hydroxy and C₁₋₆ alkoxy.

The expression "C₁₋₆ alkyl" includes methyl and ethyl groups, and straight-chained, branched or cyclic propyl, butyl, pentyl and hexyl groups. Particular alkyl groups are methyl, ethyl, n-propyl, isopropyl and tert-butyl. Derived expressions such as "C₁₋₆ alkoxy", "C₁₋₆ alkylthio" and "C₁₋₆ alkylamino" are to be construed accordingly.

The expression "C₂₋₆ alkenyl" as used herein refers to straight-chained and branched alkenyl groups containing from 2 to 6 carbon atoms. Typical examples include vinyl, allyl, dimethylallyl and butenyl groups.

The expression "C₂₋₆ alkynyl" as used herein refers to straight-chained and branched alkynyl groups containing from 2 to 6 carbon atoms. Typical examples include ethynyl and propargyl groups.

The term "aryl" refers to an aromatic ring system (monocyclic or bicyclic, only one ring needs to be aromatic) having from 6 to 10 ring carbon atoms. Typical aryl groups include phenyl and naphthyl.

The expression "aryl(C₁₋₆)alkyl" as used herein includes benzyl, phenylethyl, phenylpropyl and naphthylmethyl.

Suitable heterocycloalkyl groups include azetidinyl, pyrrolidinyl, piperidinyl, piperazinyl and morpholinyl groups.

The term "heteroaryl" refers to an aromatic ring system (monocyclic or bicyclic, only one ring needs to be aromatic) having from 5 to 10 ring atoms, in which one or more of the rings atoms are heteroatoms, such as nitrogen, sulphur, and oxygen, and the remainder are carbon atoms. Suitable heteroaryl groups include pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl and tetrazolyl groups.

The term "halogen" as used herein includes fluorine, chlorine, bromine and iodine, especially chlorine or fluorine.

For use in medicine, the salts of the compounds of formula II will be pharmaceutically acceptable salts. Other salts may, however, be useful in the preparation of the compounds of formula II or of their pharmaceutically acceptable salts. Suitable pharmaceutically acceptable salts of the compounds of formula II include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of formula II carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

Certain compounds according to the present invention may be capable of existing as tautomeric forms. It is to be understood that all possible tautomers and mixtures thereof in any proportion are encompassed within the scope of the present invention.

Where the compounds according to the invention have at least one asymmetric center, they may accordingly exist as enantiomers. Where the compounds according to the invention possess two or more asymmetric centers, they may additionally exist as diastereoisomers. It is to be understood that all such isomers and mixtures thereof in any proportion are encompassed within the scope of the present invention.

In formula II, suitable separate identities for R¹ and R² are hydrogen, and methyl, and suitable identities for R¹ and R² in combination include pyrrolidinyl, piperidinyl, piperazinyl, and 4-methylpiperazinyl.

Suitable identities for R³ include hydrogen, methyl, ethyl, benzyl, allyl, propargyl, benzoyl, phenyl, thienyl, furoyl and ethoxycarbonyl.

Suitable identities for R⁴ include benzenesulphonyl, 2-naphthalenesulphonyl, o-, m- or p-toluenesulphonyl, o-, m- or p-chlorobenzenesulphonyl, o-, m- or p-methoxy benzenesulphonyl, methanesulphonyl, dimethylaminosulphonyl, thienylsulphonyl, benzoyl, acetyl, furoyl and tert-butoxycarbonyl.

Suitable identities for R⁵ include hydroxy, methoxy, ethoxy, propoxy, benzyloxy, nitrile, fluorine, chlorine and bromine. Preferably, there is no more than one R⁵ substituent (i.e. q is 0 or 1), and when a single R⁵ substituent is present, it is preferably in the para-position relative to the indole nitrogen.

In the compounds of Formula II, R¹ and R² are identical and represent hydrogen or methyl, or together complete a pyrrolidinyl, piperidinyl, piperazinyl or 4-methylpiperazinyl ring; R³ preferably represents hydrogen or methyl; R⁴ preferably represents arylsulphonyl, thienylsulphonyl, benzoyl or tert-butoxycarbonyl; R⁵ preferably represents hydroxy, methoxy, benzyloxy or nitrile; and q is zero or 1. A sub-class of compounds in accordance with Formula II is defined by Formula II(a): 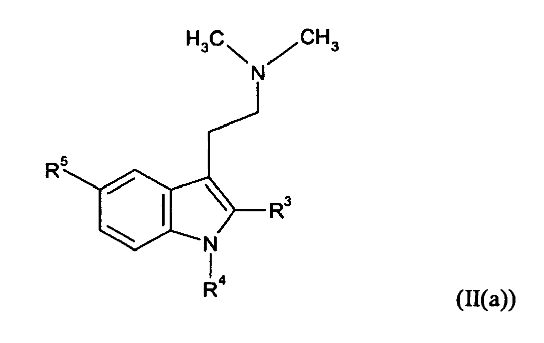 where R³, R⁴ and R⁵ have the same meanings as before.

Specific examples of compounds in accordance with Formula II(a) include:
N,N-dimethyl 2-[1-(benzenesulphonyl)-5-methoxy-1H-indol-3-yl]ethylamine;
N,N-dimethyl 2-[5-methoxy-1-(4-methyl benzenesulphonyl)-1H-indol-3-yl]ethylamine;
N,N-dimethyl 2-[1-(4-chlorobenzenesulphonyl)-5-methoxy- 1H-indol-3yl]ethylamine;
N,N-dimethyl 2-[1-(3-chlorobenzenesulphonyl)-5-methoxy-1H-indol-3-yl]ethylamine;
N,N-dimethyl 2-[5-methoxy-1-(2-naphthalenesulphonyl)-1 H-indol-3-yl]ethylamine;
N,N-dimethyl 2-[5-methoxy-1-(4-methoxybenzenesulphonyl)-1H-indol-3-yl]ethylamine;
N,N-dimethyl 2-[1-(2-chlorobenzenesulphonyl)-5-methoxy-1 H-indol-3-yl]ethylamine;
N,N-dimethyl 2-(1-benzoyl-5-methoxy-1H-indol-3-yl)ethylamine;
N,N-dimethyl 2-[5-methoxy-1-(2-thiophenesulphonyl)-1H-indol-3-yl]ethylamine;
N,N-dimethyl 2-[(1-benzenesulphonyl)-5-methoxy-2-methyl-1H-indol-3-yl]ethylamine;
N,N-dimethyl 2-(1-benzenesulphonyl-1H-indol-3-yl)ethylamine;
N,N-dimethyl 2-(1-methylsulphonyl-1H-indol-3-yl)ethylamine;
N,N-dimethyl 2-(5-methoxy-1-methylsulphonyl-1H-indol-3-yl)ethylamine;
3-(2-dimethylamino-ethyl)-5-hydroxy-1H-indole-1-carboxylic acid tert-butyl ester;
N,N-dimethyl 2-[(1-benzenesulphonyl)-5-benzyloxy-1H-indol-3-yl)]ethylamine;
N,N-dimethyl 2-[(1-benzenesulphonyl)-5-hydroxy-1H-indol-3-yl)]ethylamine;
   and
N,N-dimethyl 2-[(1-benzenesulphonyl)-5-cyano-1H-indol-3-yl)]ethylamine.

A further sub-class of compounds in accordance with Formula II is defined by Formula II(b): 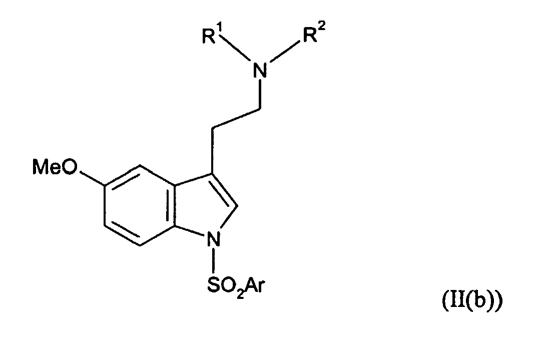 where R¹ and R² have the same meanings as before, and Ar represents an aryl or heteroaryl group.

Specific examples of compounds in accordance with Formula II(b) include:
2-[1-(benzenesulphonyl)-5-methoxy-1 H-indol-3-yl]ethylamine;
1-benzenesulphonyl-5-methoxy-3-[(2-pyrrolidin-1-yl)ethyl]-1H-indole;
1-benzenesulphonyl-5-methoxy-3-[(2-piperidin-1-yl)ethyl]-1H-indole; and
1-benzenesulphonyl-5-methoxy-3-[(2-piperazin-1-yl)ethyl]-1H-indole.

A third sub-class of compounds in accordance with Formula II is defined by Formula II(c): 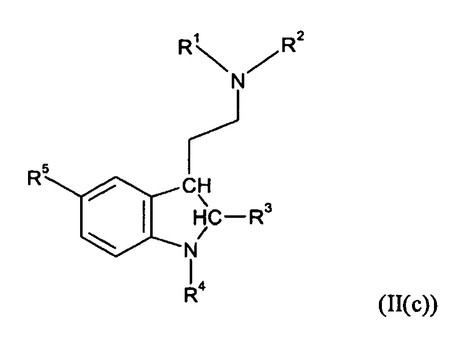 where R¹ -R⁵ have the same meanings as before.

Specific examples of compounds in accordance with Formula II(c) include:
N,N-dimethyl 2-(1-benzenesulphonyl-5-methoxy-2,3-dihydro-1H-indol-3-yl)ethylamine.

The compounds of formula II, to be used according to the invention, can be prepared according to the methods described in US 6,187,805 and GB 2,341,549.

The present invention relates to the use of compounds of Formula II, as described herein, in the manufacture of a medicament for the treatment of obesity

"An effective amount" refers to an amount of a compound that confers a therapeutic effect on the treated subject. The therapeutic effect may be objective (i.e., measurable by some test or marker) or subjective (i.e., subject gives an indication of or feels an effect). The dose level of the compounds described above, and the frequency of dosage of the specific combination, will vary depending on a variety of factors including the potency of each specific compound employed, the metabolic stability and length of action of that compound, the patient's age, body weight, general health, sex, diet, mode and time of administration, rate of excretion, drug combination, the severity of the condition to be treated, and the patient undergoing therapy.

The compounds discussed above can be brought into suitable galenic forms, such as compositions for oral use, for injection, for nasal spray administration or the like, in accordance with accepted pharmaceutical procedures. Such pharmaceutical compositions according to the invention comprise an effective amount of one, or optionally more, compound(s) discussed above in association with compatible pharmaceutically acceptable carrier materials, or diluents, as are well known in the art. The carriers may be any inert material, organic or inorganic, suitable for oral, enteral, rectal, percutaneous, subcutaneous or parenteral administration, such as: water, gelatin, gum arabicum, lactose, microcrystalline cellulose, starch, sodium starch glycolate, calcium hydrogen phosphate, magnesium stearate, talcum, colloidal silicon dioxide, and the like. Such compositions may also contain other pharmacologically active agents, and conventional additives, such as stabilizers, wetting agents, emulsifiers, flavoring agents, buffers, and the like.

The compositions according to the invention can e.g. be made up in solid or liquid form for oral administration, such as tablets, pills, capsules, powders, syrups, elixirs, dispersible granules, cachets, suppositories and the like, in the form of sterile solutions, suspensions or emulsions for parenteral administration, sprays, e.g. a nasal spray, transdermal preparations, e.g. patches, and the like.

### Example

### Effect on food intake of N,N-Dimethyl 2-[1-(benzenesulphonyl)-5-methoxy-1H-indol-3-yl]ethylamine by single dose subcutaneous administration in ob/ob mice.

### Animals

Obese (ob/ob) mouse is selected as the primary animal model for screening as this mutant mouse consumes high amounts of food resulting in a high signal to noise ratio. To further substantiate and compare efficacy data, the effect of the compounds on food consumption is also studied in wild type (C57BL/6J) mice. The amount of food consumed during 15 hours of infusion of compounds is recorded.

Male mice (obese C57BL/6JBom-Lep^{ob} and lean wild-type C57B1/6JBom; Bomholtsgaard, Denmark) 8-9 weeks with an average body weight of 50 g (obese) and 25 g (lean) are used in all the studies. The animals are housed singly in cages at 23±1°C, 40-60 % humidity and have free access to water and standard laboratory chow. The 12/12-h light/dark cycle is set to lights off at 5 p.m. The animals are conditioned for at least one week before start of study.

### Compound

The test compound, N,N-Dimethyl 2-[1-(benzenesulphonyl)-5-methoxy-1H-indol-3-yl]ethylamine, was dissolved in 25% polyethylene glycol 400 (PEG 400) plus 0,1% Tween 80 plus sodium acetate until pH 5. Doses of 30, 50 and 50 mg kg⁻¹ were used. The purity of the test compounds is of analytical grade.

### Animal dosage

| **Sex, Strain and Species:** | Male C57BL/6J-Lep^{ob}/Lep^{ob} (ob/ob) mouse |
|---|---|
| **Age & Weight:** | approx 10 weeks (- 45 gram) |
| **Route:** | sc |
| **Dose (mg salt/kg):** | 10, 30, 50 |
| **Injection volume (ml)** | 0.25 |
| **Dose volume (ml/kg):** | 5 |
| **No of administrations** | Single dose |
| **Time of administration** | 4.30 pm (lights off 5 pm) |
| **No. of animals/ treatment group:** | 8 |
| **Total no. of animals:** | 32 |

### Experimental design

The animals were divided into four groups containing 8 animals each and treated with vehicle plus three dosages of the test compound. Food consumption, total motor activity and water consumption were measured continuously for 22 h following start of recording in a computer-assisted operant test cage system (Eater meter). The animals were habituated for two days. The third day was defined as the day before treatment (basal). On the fourth day the animals were treated with test compound just before dark onset (5 pm) and data recorded cumulatively for 3 h, 6 h, 12 h and 21 h. Water consumption during 22 h was also measured by weighing the days before and after treatment.

### Statistical evaluation

Animals were randomized according to body weight and treatment assigned in a cage- and room-wise order. The food intake data are corrected for spillage during the test period of 22 h. Spillage at other time points were calculated proportionally to that of the 22 h spillage. The values are expressed as mean ± SEM both as the change in gram from basal level and as % of basal level. Statistical evaluation was performed on the percentage basal values using Kruskal-Wallis one-way ANOVA and, if significant, followed by Mann-Whitney U-test for test of significance between treatment groups. ID₂₀ values (mg salt/kg) are estimated by visual inspection and indicates the dose causing 20% inhibition of response.

### Results

N,N-Dimethyl 2-[1-(benzenesulphonyl)-5-methoxy-1H-indol-3-yl]ethylamine decreases food intake significantly at 6 h and 12 h following administration by approximately 15-20 %, see Figure 1. This effect was not dose-dependent since all doses overlapped. Total activity was not affected.

Approximate minimum effective dose 10 mg/kg (borderline statistical significance of 15-20% inhibition) for food intake.

## Claims

1. Use of a compound, which binds selectively to 5-HT₆-receptors, having a structure in accordance with Formula II: 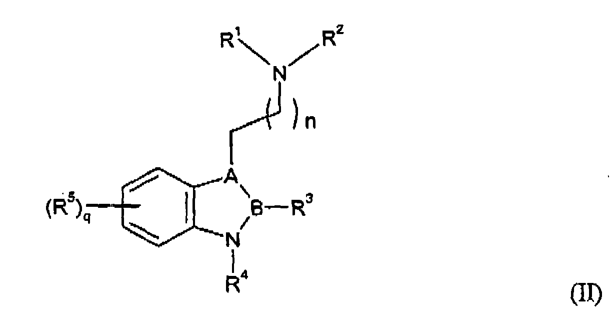 wherein
n is 1 or 2;
q is 0, 1, 2, 3 or 4;
R¹ and R² are identical and represent hydrogen or methyl, or together complete a pyrrolidinyl, piperidinyl, piperazinyl or 4-methylpiperazinyl ring comprising the nitrogen atom to which R¹ and R² are attached;
R³ represents hydrogen, C₁₋₆ alkyl, C₂₋₆ alkenyl, C₂₋₆ alkynyl, aryl(C₁₋₆)alkyl, aryl, heteroaryl, arylcarbonyl, heteroarylcarbonyl, C₁₋₆ alkylcarbonyl, or (C₁₋₆)alkoxycarbonyl, cyclopropyl, cyclobutyl, cyclopentyl, cyclohexyl, arylcyclopropyl, arylcyclobutyl, arylcyclopentyl, arylcyclohexyl, cyclopropylcarbonyl, cyclobutylcarbonyl, cyclopentylcarbonyl, cyclohexylcarbonyl, cyclopropyloxycarbonyl, cyclobutyloxycarbonyl, cyclopentyloxycarbonyl or cyclohexyloxycarbonyl;
R⁴ represents arylsulphonyl, heteroarylsulphonyl, C₁₋₆ alkylsulphonyl, di(C₁₋₆)alkylaminosulphonyl, arylcarbonyl, C₁₋₆ alkylcarbonyl, heteroarylcarbonyl or C₁₋₆ alkoxycarbonyl, cyclopropylsulphonyl, cyclobutylsulphonyl, cyclopentylsulphonyl, cyclohexylsulphonyl, di-cyclopropylaminosulphonyl, di-cyclobutylaminosulphonyl, di-cyclopentylaminosulphonyl, di-cyclohexylaminosulphonyl, cyclopropylcarbonyl, cyclobutylcarbonyl, cyclopentylcarbonyl, cyclohexylcarbonyl, cyclopropyloxycarbonyl, cyclobutyloxycarbonyl, cyclopentyloxycarbonyl or cyclohexyloxycarbonyl;
each R⁵ independently represents hydrogen, hydroxy, C₁₋₆ alkoxy, aryl(C₁₋₆)alkoxy, nitrile or halogen, cyclopropyloxy, cyclobutyloxy, cyclopentyloxy, cyclohexyloxy, arylcyclopropyloxy, arylcyclobutyloxy, arylcyclopentyloxy, arylcyclohexyloxy; and
A-B represents C=C or CH-CH,
in the manufacture of a medicament for the treatment and/or prevention of obesity.

2. Use according to claim 1 in which said compound is in accordance with said Formula II wherein
R¹ and R² are identical and represent hydrogen or methyl, or together complete a pyrrolidinyl, piperidinyl, piperazinyl or 4-methylpiperazinyl ring with the nitrogen atom to which R¹ and R² are attached;
R³ represents hydrogen, methyl, ethyl, benzyl, allyl, propargyl, benzoyl, phenyl, thienyl, furoyl, or ethoxycarbonyl;
R⁴ represents benzenesulphonyl, 2-naphthalenesulphonyl, o-, m- or p-toluenesulphonyl, o-, m- or p-chlorobenzenesulphonyl, o-, m- or p-methoxybenzenesulphonyl, methanesulphonyl, dimethylaminosulphonyl, thienylsulphonyl, benzoyl, acetyl, furoyl or tert-butoxycarbonyl; and
R⁵ represents hydrogen, hydroxy, methoxy, ethoxy, propoxy, benzyloxy, nitrile, fluorine, chlorine or bromine.

3. Use according to claim 1 in which the compound is selected from:
compounds of Formula II in which R¹ and R² are identical and represent hydrogen or methyl, or together complete a pyrrolidinyl, piperidinyl, piperazinyl or 4-methylpiperazinyl ring : R³ represents hydrogen or methyl; R⁴ represents arylsulphonyl, thienylsulphonyl, benzoyl or tert-butoxycarbonyl; R⁵ represents, hydroxy, methoxy, benzyloxy or nitrile; and q is zero or 1.

4. Use according to claim 1 in which the compound is in accordance with Formula II(a): 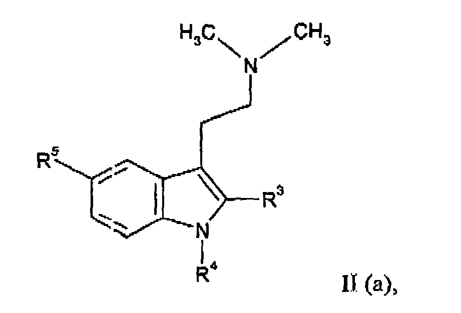 wherein R³, R⁴, and R⁵ are as defined in claim 1.

5. Use according to claim 4 in which the compound is:
N,N-dimethyl 2-1-(benzenesulphonyl)-5-methoxy-1H-indol-3-yl]-ethylamine;
N,N-tlimethyl 2-[5-methoxy-1-(4-methyl benzenesulphonyl)-1H-indol-3-yl]ethylamine ;
N,N-dimethyl 2-[1-(4-chlorobenzenesulphonyl)-5-methoxy- 1H-indol-3yl]ethylamine ;
N,N-dimethyl 2-[1-(3-chlorobenzenesulphonyl)-5-methoxy-1H-indol-3-yl]ethylamine;
N,N-dimethyl 2-[5-methoxy-1-(2-naphthalenesulphonyl)-1H-indol-3-yl]ethylamine;
N,N-dimethyl 2-[5-methoxy-1-(4-methoxybenzenesulphonyl)-1H-indol-3-yl]ethylamine;
N,N-dimethyl 2-[1-(2-chlorobenzenesulphonyl)-5-methoxy-1H-indol-3-yl]ethylamine;
N,N-dimethyl 2-(1-benzoyl-5-methoxy-1H-indol-3-yl)ethylamine;
N,N-dimethyl 2-[5-methoxy-1-(2-thiophenesulphonyl)-1H-indol-3-yl]ethylamine;
N,N-dimethyl 2-[(1-benzenesulphonyl)-5-methoxy-2-methyl-1H-indol-3-yl]ethylamine;
N,N-dimethyl 2-(1-benzenesulphonyl-1H-indol-3-yl)ethylamine;
N,N-dimethyl 2-(1-methylsulphonyl-1 H-indol-3-yl)ethylamine;
N,N-dimethyl 2-(5-methoxy-1-methylsulphonyl-1H-indol-3-yl)ethylamine;
3-(2-dimethylamino-ethyl)-5-hydroxy-1H-indole-1-carboxylic acid tert-butyl ester;
N,N-dimethyl 2-[(1-benzenesulphonyl)-5-benzyloxy-1H-indol-3-yl)]ethylamine;
N,N-dimethyl 2-[(1-benzenesulphonyl)-5-hydroxy-1H-indol-3-yl)]ethylamine; or
N,N-dimetlayl 2-[(1-benzenesulphonyl)-5-cyano-1H-indol-3-yl)]éthylamine.

6. Use according to claim 1 in which the compound is in accordance with formula II(b): 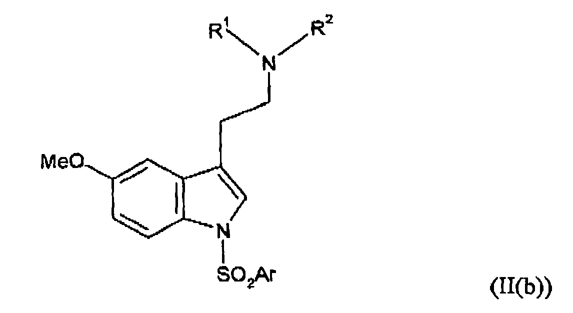 wherein R¹ and R² are as defined in claim 1, and Ar represents an aryl or heteroaryl group.

7. Use according to claim 6, wherein R¹ and R² are methyl groups.

8. Use according to any one of claim 6 or 7, wherein Ar is selected from the group consisting of phenyl, 2-thienyl, and 3-chlorophenyl.

9. Use according to claim 6, wherein the compound is:
2-[1-(benzenesulphonyl)-5-methoxy-1H-indol-3-yl]ethylamine;
1-benzenesulphonyl-5-methoxy-3-[(2-pyrrolidin-1-yl)ethyl]-1H-indole;
1-benzenesulphonyl-5-methoxy-3-[(2-piperidin-1-yl)ethyl]-1H-indole; or
1-benzenesulphonyl-5-methoxy-3-[(2-piperazin-1-yl)ethyl]-1H-indole.

10. Use according to claim 1 in which the compound is in accordance with Formula II(c): 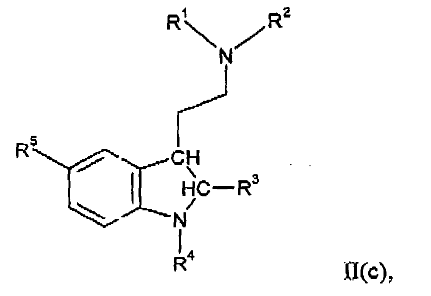 wherein R¹, R², R³, R⁴, and R⁵ are as defined in claim 1.

11. Use according to claim 10, wherein the said compound is N,N-dimethyl-2-[1-(benzenesulphonyl)-5-methoxy-2,3-dihydro-1H-indol-3-yl]ethylamine,

## Patentansprüche

1. Verwendung einer Verbindung, welche selektiv an 5-HT₆-Rezeptoren bindet, mit einer Struktur gemäß Formel II: 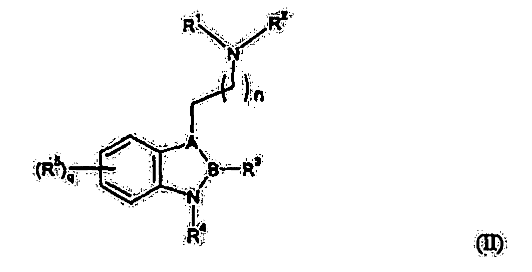 worin
n 1 oder 2 ist;
q 0, 1, 2, 3 oder 4 ist;
R¹ und R² identisch sind und für Wasserstoff oder Methyl stehen, oder zusammen einen Pyrrolidinyl-, Piperidinyl-, Piperazinyl- oder 4-Methylpiperazinylring vervollständigen, der das Stickstoffatom umfasst, an welchem R¹ und R² gebunden sind;
R³ für Wasserstoff, C₁₋₆-Alkyl, C₂₋₆-Alkenyl, C₂₋₆-Alkinyl, Aryl(C₁-₆)alkyl, Aryl, Heteroaryl, Arylcarbonyl, Heteroarylcarbonyl, C₁₋₆-Alkylcarbonyl oder (C₁₋₆)Alkoxycarbonyl, Cyclopropyl, Cyclobutyl, Cyclopentyl, Cyclohexyl, Arylcyclopropyl, Arylcyclobutyl, Arylcyclopentyl, Arylcyclohexyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl oder Cyclohexyloxycarbonyl steht;
R⁴ für Arylsulfonyl, Heteroarylsulfonyl, C₁₋₆-Alkylsulfonyl, Di(C₁-₆)alkylaininosulfonyl, Arylcarbonyl, C₁₋₆-Alkylcarbonyl, Heteroarylcarbonyl oder C₁₋₆-Alkoxycarbonyl, Cyclopropylsulfonyl, Cyclobutylsulfonyl, Cyclopentylsulfonyl, Cyclohexylsulfonyl, Dicyclopropylaminosulfonyl, Di-cyclobutylaminosulfonyl, Di-cyclopentylaminosulfonyl, Di-cyclohexylaminosulfonyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl oder Cyclohexyloxycarbonyl steht;
jedes von R⁵ unabhängig für Wasserstoff, Hydroxy, C₁₋₆-Alkoxy, Aryl(C₁₋₆)alkoxy, Nitril oder Halogen, Cyclopropyloxy, Cyclobutyloxy, Cyclopentyloxy, Cyclohexyloxy, Arylcyclopropyloxy, Arylcyclobutyloxy, Arylcyclopentyloxy, Arylcyclohexyloxy steht; und
A-B für C=C oder CH-CH steht,
bei der Herstellung eines Medikaments zur Behandlung und/oder Vorbeugung von Fettleibigkeit.

2. Verwendung gemäß Anspruch 1, wobei die Verbindung in Übereinstimmung mit der Formel II ist, worin
R¹ und R² identisch sind und für Wasserstoff oder Methyl stehen, oder zusammen einen Pyrrolidinyl-, Piperidinyl-, Piperazinyl- oder 4-Methylpiperazinylring mit dem Stickstoffatom vervollständigen, an welchem R¹ und R² gebunden sind;
R³ für Wasserstoff, Methyl, Ethyl, Benzyl, Allyl, Propargyl, Benzoyl, Phenyl, Thienyl, Furoyl oder Ethoxycarbonyl steht;
R⁴ für Benzolsulfonyl, 2-Naphthalinsulfonyl, o-, m- oder p-Toluolsulfonyl, o-, m- oder p-Chlorbenzolsulfonyl, o-, m- oder p-Methoxybenzolsulfonyl, Methansulfonyl, Dimethylaminosulfonyl, Thienylsulfonyl, Benzoyl, Acetyl, Furoyl oder tert-Butoxycarbonyl steht; und
R⁵ für Wasserstoff, Hydroxy, Methoxy, Ethoxy, Propoxy, Benzyloxy, Nitril, Fluor, Chlor oder Brom steht.

3. Verwendung gemäß Anspruch 1, wobei die Verbindung gewählt wird aus:
Verbindungen der Formel II, worin R¹ und R² identisch sind und für Wasserstoff oder Methyl stehen, oder zusammen einen Pyrrolidinyl, Piperidinyl, Piperazinyl oder 4-Methylpiperazinylring bilden; R³ für Wasserstoff oder Methyl steht; R⁴ für Arylsulfonyl, Thienylsulfonyl, Benzoyl oder tert-Butoxycarbonyl steht; R⁵ für Hydroxy, Methoxy, Benzyloxy oder Nitril steht; und q Null oder 1 ist.

4. Verwendung gemäß Anspruch 1, wobei die Verbindung in Übereinstimmung mit der Formel II(a) ist: 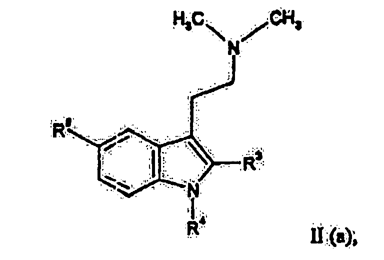 worin R³, R⁴ und R⁵ wie in Anspruch 1 definiert sind.

5. Verwendung gemäß Anspruch 4, wobei die Verbindung Folgendes ist:
N,N-Dimethyl-2-[1-(benzolsulfonyl)-5-methoxy-1H-indol-3-yl]ethylamin ;
N,N-Dimethyl-2-[5-methoxy-1-(4-methylbenzolsulfonyl)-1 H-indol-3-yl]ethylamin;
N,N-Dimethyl-2-[1-(4-chlorbenzolsulfonyl)-5-methoxy-1H-indol-3yl]ethylamin;
N,N-Dimethyl-2-[1-(3-chlorbenzolsulfonyl)-5-methoxy-1H-indol-3-yl]ethylamin;
N,N-Dimethyl-2-[5-methoxy-1-(2-naphthalinsulfonyl)-1H-indol-3-yl]ethylamin ;
N,N-Dimethyl-2-[5-methoxy-1-(4-methoxybenzolsulfonyl)-1H-indol-3-yl]ethylamin;
N,N-Dimethyl-2-[1-(2-chlorbenzolsulfonyl)-5-methoxy-1H-indol-3-yl]ethylamin;
N,N-Dimethyl-2-(1-benzoyl-5-methoxy-1H-indol-3-yl] ethylamin;
N,N-Dimethyl-2-[5-methoxy-1-(2-thiophensulfonyl)-1 H-indol-3-yl] ethylamin;
N,N-Dimethyl-2-[(1-benzolsulfonyl)-5-methoxy-2-methyl-1H-indol-3-yl]ethylamin;
N,N-Dimethyl-2-(1-benzolsulfonyl-1H-indol-3-yl)ethylamin;
N,N-Dimethyl-2-(1-methylsulfonyl-1 H-indol-3-yl)ethylamin;
N,N-Dimethyl-2-(5-methoxy-1-methylsulfonyl-1 H-indol-3-yl)ethylamin;
3-(2-Dimethylamino-ethyl)-5-hydroxy-1H-indol-1-carbonsäure-tert-butylester;
N,N-Dimethyl-2-[(1-benzolsulfonyl)-5-benzyloxy-1H-indol-3-yl)]ethylamin;
N,N-Dimethyl-2-[(1-benzolsulfonyl)-5-hydroxy-1H-indol-3-yl)]ethylamin; oder
N,N-Dimethyl-2-[(1-benzolsulfonyl)-5-cyano-1H-indol-3-yl)]ethylamin.

6. Verwendung gemäß Anspruch 1, wobei die Verbindung in Übereinstimmung mit der Formel II(b) ist: 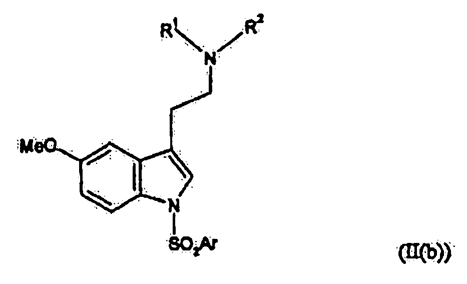 worin R¹ und R² wie in Anspruch 1 definiert sind und Ar für eine Aryl- oder Heteroarylgruppe steht.

7. Verwendung gemäß Anspruch 6, wobei R¹ und R² Methylgruppen sind.

8. Verwendung gemäß mindestens einem der Ansprüche 6 oder 7, wobei Ar aus der Gruppe gewählt wird, die aus Phenyl, 2-Thienyl und 3-Chlorphenyl besteht.

9. Verwendung gemäß Anspruch 6, wobei die Verbindung Folgendes ist:
2-[1-(Benzolsulfonyl)-5-methoxy-1H-indol-3-yl]ethylamin ;
1-Benzolsulfonyl-5-methoxy-3-[(2-pyrrolidin-1-yl)ethyl]-1 H-indol;
1-Benzolsulfonyl-5-methoxy-3-[(2-piperidin-1-yl)ethyl]-1H-indol; oder
1-Benzolsulfonyl-5-methoxy-3-[(2-piperazin-1-yl)ethyl]-1H-indol.

10. Verwendung gemäß Anspruch 1, wobei die Verbindung in Übereinstimmung mit der Formel II(c) ist: 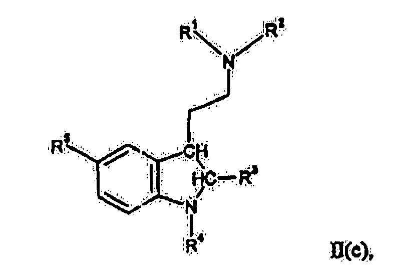 worin R¹, R², R³, R⁴ und R⁵ wie in Anspruch 1 definiert sind.

11. Verwendung gemäß Anspruch 10, wobei die Verbindung N,N-Dimethyl-2-[1-benzolsulfonyl)-5-methoxy-2,3-dihydro-1H-indol-3-yl]ethylamin ist.

## Revendications

1. Utilisation d'un composé qui se lie sélectivement aux récepteurs de 5-HT₆, ayant une structure de formule II: 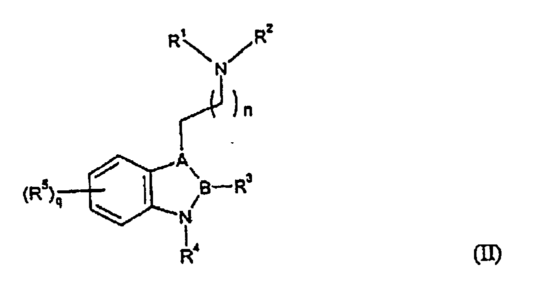 dans laquelle
n est 1 ou 2;
q est 0, 1, 2, 3 ou 4;
R¹ et R² sont identiques et représentent un atome d'hydrogène ou un méthyle, ou complètent ensemble un cycle pyrrolidinyle, pipéridinyle, pipérazinyle ou 4-méthylpipérazinyle comprenant l'atome d'azote auquel sont liés R¹ et R²;
R³ représente un atome d'hydrogène, un alkyle en C₁-C₆, un alcényle en C₂-C₆, un alcynyle en C₂-C₆, un aryl(alkyle en C₁-C₆), un aryle, un hétéroaryle, un arylcarbonyle, un hétéroarylcarbonyle, un (alkyle en C₁-C₆)carbonyle, ou un (alcoxy en C₁-C₆)-carbonyle, un cyclopropyle, un cyclobutyle, un cyclopentyle, un cyclohexyle, un arylcyclopropyle, un arylcyclobutyle, un arylcyclopentyle, un arylcyclohexyle, un cyclopropylcarbonyle, un cyclobutylcarbonyle, un cyclopentylcarbonyle, un cyclohexylcarbonyle, un cyclopropyloxycarbonyle, un cyclobutyloxycarbonyle, un cyclopentyloxycarbonyle ou un cyclohexyl-oxycarbonyle;
R⁴ représente un arylsulfonyle, un hétéroarylsulfonyle, un alkylsulfonyle en C₁-C₆, un di(alkyle en C₁-C₆)aminosulfonyle, un arylcarbonyle, un (alkyle en C₁-C₆)carbonyle, un hétéroarylcarbonyle ou un (alcoxy en C₁-C₆)carbonyle, un cyclopropylsulfonyle, un cyclobutylsulfonyle, un cyclopentylsulfonyle, un cyclohexylsulfonyle, un dicyclopropylaminosulfonyle, un dicyclobutylamino-sulfonyle, un dicyclopentylaminosulfonyle, un dicyclohexylaminosulfonyle, un cyclopropylcarbonyle, un cyclobutylcarbonyle, un cyclopentylcarbonyle, un cyclohexylcarbonyle, un cyclopropyloxycarbonyle, un cyclobutyloxycarbonyle, un cyclopentyloxycarbonyle ou un cyclohexyloxycarbonyle;
chaque R⁵ représente indépendamment un atome d'hydrogène, un hydroxy, un alcoxy en C₁-C₆, un aryl-(alcoxy en C₁-C₆), un nitrile ou un halogène, un cyclopropyloxy, un cyclobutyloxy, un cyclopentyloxy, un cyclohexyloxy, un arylcyclopropyloxy, un arylcyclobutyloxy, un arylcyclopentyloxy, un arylcyclohexyloxy; et
A-B représente C=C ou CH-CH,
dans la fabrication d'un médicament destiné au traitement et/ou à la prévention de l'obésité.

2. Utilisation selon la revendication 1, dans laquelle ledit composé est conforme à ladite formule II dans laquelle
R¹ et R² sont identiques et représentent un atome d'hydrogène ou un méthyle, ou complètent ensemble un cycle pyrrolidinyle, pipéridinyle, pipérazinyle ou 4-méthylpipérazinyle avec l'atome d'azote auquel sont liés R¹ et R²;
R³ représente un atome d'hydrogène, un méthyle, un éthyle, un benzyle, un allyle, un propargyle, un benzoyle, un phényle, un thiényle, un furoyle ou un éthoxycarbonyle;
R⁴ représente un benzènesulfonyle, un 2-naphtalènesulfonyle, un o-, m- ou p-toluènesulfonyle, un o-, m- ou p-chlorobenzènesulfonyle, un o-, m- ou p-méthoxybenzènesulfonyle, un méthanesulfonyle, un diméthylaminosulfonyle, un thiénylsulfonyle, un benzoyle, un acétyle, un furoyle ou un tert-butoxycarbonyle; et
R⁵ représente un atome d'hydrogène, un hydroxy, un méthoxy, un éthoxy, un propoxy, un benzyloxy, un nitrile, un fluor, un chlore ou un brome.

3. Utilisation selon la revendication 1, dans laquelle le composé est choisi parmi des composés de formule II dans laquelle R¹ et R² sont identiques et représentent un atome d'hydrogène ou un méthyle, ou complètent ensemble un cycle pyrrolidinyle, pipéridinyle, pipérazinyle ou 4-méthylpipérazinyle; R³ représente un atome d'hydrogène ou un méthyle, R⁴ représente un arylsulfonyle, un thiénylsulfonyle, un benzoyle ou un tert-butoxycarbonyle; R⁵ représente un hydroxy, un méthoxy, un benzyloxy ou un nitrile; et q est 0 ou 1.

4. Utilisation selon la revendication 1, dans laquelle le composé est conforme à la formule II(a): 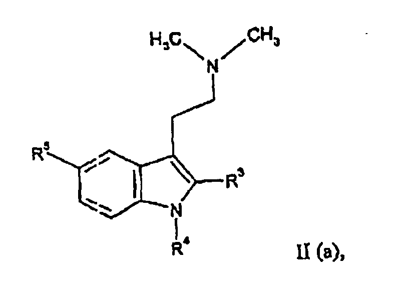 dans laquelle R³, R⁴ et R⁵ sont tels que définis dans la revendication 1.

5. Utilisation selon la revendication 4, dans laquelle le composé est:
la N,N-diméthyl-2-[1-(benzènesulfonyl)-5-méthoxy-1H-indol-3-yl]éthylamine;
la N,N-diméthyl-2-[5-méthoxy-1-(4-méthylbenzènesulfonyl)-1H-indol-3-yl]éthylamine;
la N,N-diméthyl-2-[1-(4-chlorobenzènesulfonyl)-5-méthoxy-1H-indol-3-yl]éthylamine;
la N,N-diméthyl-2-[1-(3-chlorobenzènesulfonyl)-5-méthoxy-1H-indol-3-yl]éthylamine;
la N,N-diméthyl-2-[5-méthoxy-1-(2-naphthalènesulfonyl)-1H-indol-3-yl]éthylamine;
la N,N-diméthyl-2-[5-méthoxy-1-(4-méthoxybenzènesulfonyl)-1 H-indol-3yl]éthylamine;
la N,N-diméthyl-2-[1-(2-chlorobenzènesulfonyl)-5-méthoxy-1H-indol-3- yl]éthylamine;
la N,N-diméthyl-2-(1-benzoyl-5-méthoxy-1H-indol-3-yl) éthylamine;
la N,N-diméthyl-2-[5-méthoxy-1-(2-thiophènesulfonyl)-1H-indol-3-yl]éthylamine;
la N,N-diméthyl-2-[(1-benzènesulfonyl)-5-méthoxy-2-méthyl-1H-indol-3-yl]éthylamine;
la N,N-diméthyl-2-(1-benzènesulfonyl-1H-indol-3 -yl)éthylamine;
la N,N-diméthyl-2-(1-méthylsulfonyl-1H-indol-3-yl)éthylamine;
la N,N-diméthyl-2-(5-méthoxy-1-méthylsulfonyl-1H-indol-3-yl)éthylamine;
le 3-(2-diméthylaminoéthyl)-5-hydroxy-1H-indole-1-carboxylate de tert-butyle;
la N,N-diméthyl-2-[(1-benzènesulfonyl)-5-benzyloxy-1H-indol-3-yl)]éthylamine;
la N,N-diméthyl-2-[(1-benzènesulfonyl)-5-hydroxy-1H-indol-3-yl)]éthylamine; ou
la N,N-diméthyl-2-[(1-benzènesulfonyl)-S-cyano-1H-indol-3-yl)]éthylamine.

6. Utilisation selon la revendication 1, dans lequel le composé est conforme à la formule II(b): 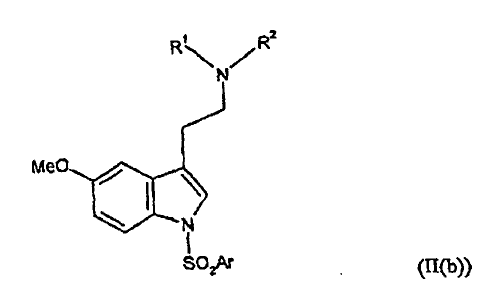 dans laquelle R¹ et R² sont tels que définis dans la revendication 1, et Ar représente un groupe aryle ou hétéroaryle.

7. Utilisation selon la revendication 6, dans laquelle R¹ et R² sont des groupes méthyle.

8. Utilisation selon l'une quelconque des revendications 6 ou 7, dans laquelle Ar est choisi dans le groupe constitué par phényle, 2-thiényle et 3-chlorophényle.

9. Utilisation selon la revendication 6, dans laquelle le composé est:
la 2-[1-benzènesulfonyl)-5-méthoxy-1H-indol-3-yl]éthylamine;
le 1-benzènesulfonyl-5-méthoxy-3-[(2-pyrrolidin-1-yl)éthyl]-1H-indole;
le 1-benzènesulfonyl-5-méthoxy-3-[(2-pipéridin-1-yl)éthyl]-1H-indole ; ou
le 1-benzènesulfonyl-5-méthoxy-3-[(2-pipérazin-1-yl)éthyl]-1H-indole.

10. Utilisation selon la revendication 1, dans laquelle le composé est conforme à la formule II(c): 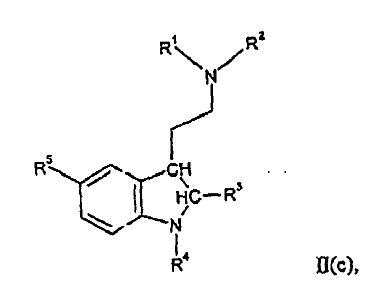 dans laquelle R¹, R², R³, R⁴ et R⁵ sont tels que définis dans la revendication 1.

11. Utilisation selon la revendication 10, dans laquelle ledit composé est la N,N-diméthyl-2-[1-(benzènesulfonyl)-5-méthoxy-2,3-dihydro-1H-indol-3-yl]éthylamine.
